# EUROPEAN PATENT APPLICATION

(11) **EP 1 231 276 A1**
(43) Date of publication of application: **14.08.2002**
(21) Application number: 02004765.0
(22) Date of filing: 17.08.1999
(51) Int. Cl.: C12N 15/74, C12N 15/11, C12N 15/62, C12P 21/02

(54) **Method for purifying proteins and/or biomolecule of protein complexes**

(30) Priority: 17.08.1998 EP 98115448
(62) Divisional of application: 99944420.1
(71) Applicant: EUROPÄISCHES LABORATORIUM FÜR MOLEKULARBIOLOGIE (EMBL), D-69117 Heidelberg (DE)
(72) Inventor: Seraphin, Bertrand, 78114 Magny les Hameaux (FR); Rigaut, Guillaume, 69007 Lyon (FR)
(74) Representative: Dey, Michael, Dr.

(57) **Abstract**

The present invention relates to a method for detecting and/or purifying proteins and/or biomolecule or protein complexes as well as fusion proteins, nucleic acids, vectors and cells suitable for this method.

## Description

The present invention relates to a method for purifying substances such as biomolecules, proteins, protein and/or biomolecule complexes, subunits of biomolecule complexes, cell components, cell organelles or even whole cells. It also concerns fusion proteins for use in this method and other related subjects.

Protein expression and purification methods are essential for studying the structure, activities, interactions with other proteins, nucleic acids etc. of proteins of interest. Methods that are currently available use systems such as bacteria or cells transfected with expression vectors or infected with bacculovirus.

In order to study individual proteins a first requirement is to obtain sufficient amounts of that particular protein to be able to carry out biological and biochemical analyses such as activity tests, interaction assays, structure determination and the like. For this purpose the genes coding for the proteins of interest are cloned into vectors that allow the expression of those proteins in suitable host cells. Usually the proteins are expressed at high levels. This over-expression leads to the generation of large amounts of protein but often has the disadvantage of yielding insoluble protein which is present in so-called inclusion bodies in the cells. The over-expressed protein then has to be resolubilized before analysis. Although such methods work well for conventional protein detection methods based on weight analysis (polyacrylamide gels, Western blots, etc.) of the expressed protein, they are not suitable for other studies and for assays on protein complexes.

Currently used protocols for over-expressing proteins are normally carried out in host cells in which the protein of interest is not normally expressed, for example, when eukaryotic proteins are expressed in bacteria. Apart from the insolubility, proteins expressed in this manner often show a lack of proper post-transcriptional or post-translational modification, such as correct processing or glycosylation.

Another disadvantage is that the expression of one subunit of a complex, even in a homologous system, might not lead to increased production of all the complex components and in some extreme cases can even result in mistargeting of that protein of interest to an aberrant complex (e.g. the proteasome, Swafield et al., 1996, Nature 379, 658).

Purification of proteins expressed at their basal level is therefore indispensable in many cases but appropriate purification protocols are not easily available because of the huge biochemical knowledge required to establish a suitable protocol. Developing a purification scheme requires assessing the chemical and physical properties of the target protein by a trial and error process, making it tedious and time-consuming because such analyses have to be repeated for each protein.

Affinity purification methods for the purification of proteins are known. Kits and apparatuses for affinity chromatography are commercially available. Usually, a fusion protein of a polypeptide of interest plus an affinity tag is expressed in a system such as one of the above-mentioned expression systems, the fusion protein is extracted and applied to support material such as a resin matrix packed in an affinity column which is coated with a material that specifically binds the affinity tag. After binding of the target protein to the matrix via the affinity tag unbound substances can be removed simply by washing the matrix. The fusion protein can then be eluted off the matrix using chemical agents or specific temperature or pH conditions. Since the affinity tags usually bind with high affinity strong conditions are needed for the subsequent elution wich can often destroy, damage or denature the protein of interest.

Affinity tags possess groups or moieties which are capable of binding to a specific binding partner with high affinity. Various affinity tags are known in the art and have been widely used for the purification of proteins. Examples are the IgG binding domains of protein A of Staphylococcus aureus, glutathione-S-transferase (GST), maltose binding protein, cellulose binding domain, polyarginine, polycysteine, polyhistidine, polyphenylalanine, calmodulin or calmodulin binding domains. These bind with high affinity to an appropriate matrix which is covered with the specific binding partner. In the case of protein A, IgG-coated sepharose has been used for affinity chromatography of fusion proteins possessing a protein A domain (Senger et al., 1998, EMBO J., Vol.17, 2196-2207). Other examples are discussed in Sassenfeld, TIBTECH, 1990, p.88. A plasmid vector containing a cassette encoding a calmodulin binding peptide is available from Stratagene.
Normally, fusion proteins are tagged with only one affinity tag and are purified in a single purification step. This often leads to problems due to remaining contaminants. Another limitation of most of the conventional methods is that they are adapted for expression of the proteins in bacteria only. WO96/40943 discloses a method of expressing fusion proteins in gram-positive bacteria either anchored to the membrane or in secreted form. The anchored proteins are cleaved off using TEV protease and subsequently affinity purified via an affinity tag.

Often the affinity tag is removed from the fusion protein after the affinity purification step by the action of a specific protease such as the TEV protease. This means however, that the purified fractions contain substantial amounts of this protease (Senger et al. 1998) which severely limits the applications of such protein preparations.

It is therefore an object of the present invention to provide a purification/detection method for proteins and/or biomolecule or protein complexes and/or components or subunits thereof which eliminates the disadvantages of the currently known methods and allows efficient purification not only of affinity tagged target proteins as fusion proteins but also of other substances that are capable of associating with these proteins.
One method according to the invention for purifying substances selected from proteins, biomolecules, complexes of proteins or biomolecules, subunits thereof, cellular components, cell organelles, and whole cells comprises the following steps:
(a) providing an expression environment containing one or more heterologous nucleic acids encoding one or more polypeptides and/or one or more subunits of a biomolecule complex, the polypeptides or subunits being fused to at least two different affinity tags, one of which consists of one or more IgG binding domains of Staphylococcus protein A,
(b) maintaining the expression environment under conditions that facilitate expression of the one or more polypeptides or subunits in a native form as fusion proteins with the affinity tags,
(c) detecting and/or purifying the one or more polypeptides or subunits by a combination of at least two different affinity purification steps each comprising binding the one or more polypeptides or subunits via one affinity tag to a support material capable of selectively binding one of the affinity tags and separating the one or more polypeptides or subunits from the support material after substances not bound to the support material have been removed.

An alternative method of the invention which is particularly suitable for detecting and/or purifying protein or biomolecule complexes is a method comprising the steps:
(a) providing an expression environment containing one or more heterologous nucleic acids encoding at least two subunits of a biomolecule complex, each being fused to at least one of different affinity tags, one of which consists of one or more IgG binding domains of Staphylococcus protein A,
(b) maintaining the expression environment under conditions that facilitate expression of the one or more subunits in a native form as fusion proteins with the affinity tags, and under conditions that allow the formation of a complex between the one or more subunits and possibly other components capable of complexing with the one or more subunits,
(c) detecting and/or purifying the complex by a combination of at least two different affinity purification steps each comprising binding the one or more subunits via one affinity tag to a support material capable of selectively binding one of the affinity tags and separating the complex from the support material after substances not bound to the support material have been removed.

For the purpose of this invention, a biomolecule can be a protein, peptide or a nucleic acid or other biomolecule. A biomolecule complex denotes a complex of at least two biomolecules, preferably at least one protein associated either with other proteins which are then called subunits or with other substances which can for example be nucleic acids. The biomolecule complexes can be natural ones such as nuclear snRNPs or antigen-antibody complexes, or they can be artificial ones such as mutant DNA binding proteins associated with mutant target DNAs. Any complex molecule comprising as one or more subunits a polypeptide or subunit expressed according to the invention and/or further comprising other components which associate in a manner stable enough not to be dissociated by the affinity steps is a biomolecule complex that can be detected and/or purified by the method of the invention. A protein complex generally devotes a complex between protein subunits.

The nucleic acid sequence of the protein to be purified must be known or at least available so that it can be cloned into a nucleic acid which is suitable to drive expression in the appropriate host cells or cell-free expression systems. If a protein complex is to be purified, the nucleic acid sequence of at least one of its subunits has to be known or available.

The heterologous nucleic acid driving the expression of the protein to be purified according to the invention thus contains appropriate sequences that allow it to be maintained in the chosen host cell or cell-free system, such as a promoter and, if necessary, other control sequences such as enhancers and poly A sites.

In principle any host cell that is compatible with the heterologous nucleic acid from which the polypeptides or subunits are to be expressed is suitable as an expression environment. These cells can be prokaryotic cells such as bacteria etc. or eukaryotic cells such as yeast, fungi or mammalian cells. Preferably, the protein or subunit or protein complex to be purified is expressed in its natural host. Since this method is very efficient, the proteins are preferably expressed at their basal levels.This has the advantage of avoiding the formation of inclusion bodies and also reduces the risk of toxic effects on the cell that large amounts of certain proteins may have. Furthermore, this avoids purifying excess protein subunits that are not assembled into a complex or that are assembled into aberrant complexes (see above).

After the heterologous nucleic acid encoding the fusion protein has been introduced into a chosen host cell the cell is cultured under conditions which allow the expression of the fusion protein(s).

As already mentioned, the transcriptional control sequences are preferably selected so that the fusion protein is not over-expressed but is expressed at basal levels in the cell. This serves to ensure that the protein is expressed in a native form. Native form means in this context that a correct or relatively close to natural three-dimensional structure of the protein is achieved, i.e. the protein is folded correctly. More preferably, the protein will also be processed correctly and show normal post-transcriptional and post-translational modification. The correct folding is of great importance especially when the expressed polypeptide is a subunit of a protein complex because it will bind to the other subunits of the complex only when it is present in its native form. However, it is also possible to express mutant proteins. These can also have a native conformation. Such mutant subunits can, for example, be used to purify mutant complexes, i.e. complexes that contain some other mutated subunits.

Depending on the protein or subunit to be purified, the fusion protein is expressed intracellularly or secreted into the culture medium. Alternatively, it might be targeted to other cell compartments such as the membrane. Depending on the protein an appropriate method is used to extract the fusion protein from the cells and/or medium. When a fusion protein is expressed which is targetted to a certain subcellular location, e.g. the membrane of cell organelles or the cell membrane, these organelles or the cells themselves can be purified via the binding of these membrane proteins. It is also possible to purify cells or cell organelles via proteins naturally expressed on their surface which bind to the fusion protein of the invention.

Further, it is possible to purify biomolecule or protein complexes/subunits or other substances that are capable of binding to or complexing with the fusion protein generated according to the invention. These substances can bind to fusion protein either directly or via linker mediators. Linker mediators in this context may be anything which is capable of binding two or more biomolecules so that these biomolecules are then part of a complex although they may not be directly associated with each other.

According to the invention it is also possible to use cell-free systems for the expression of the polypeptides or subunits. These must provide all the components necessary to effect expression of proteins from the nucleic acid such as transcription factors, enzymes, ribosomes etc. In vitro transcription and translation systems are commercially available as kits so that it is not necessary to describe these systems in detail (e.g. rabbit reticulocyte lysate systems for translation). A cell-free or in vitro system should also allow the formation of complexes.

For the purification according to the invention it is preferable to employ affinity chromatography on affinity columns which contain a matrix coated with the appropriate binding partner for the affinity tag used in that particular purification step.

In accordance with the method of this invention two affinity steps are carried out. Basically each affinity step consists of a binding step in which the previously extracted protein is bound via one of its affinity tags to a support material which is covered with the appropriate binding partner for that affinity tag. Then unbound substances are removed and finally the protein to be purified is recovered from the support material. This can be done in two ways. The first possibility is to simply use conventional elution techniques such as varying the pH or the salt or buffer concentrations and the like depending on the tag used. The second possibility is to release the protein to be purified from the support material by proteolytically cleaving off the affinity tag bound to the support. This way, the protein can be recovered in the form of a truncated fusion protein or, if all affinity tags have been cleaved off, as the target polypeptide or subunit itself.

According to one embodiment of the present invention a fusion protein of a single polypeptide plus two different affinity tags is expressed, wherein one of the tags comprises one or more IgG binding domains of protein A of Staphylococcus aureus.

More preferably, a specific proteolytic cleavage site is present in the fusion protein between the one or more polypeptides or subunits and the one or more affinity tags so that proteolytic cleavage allows the removal of at least one of the affinity tags, especially the IgG binding domains of protein A.

Proteolytic cleavage can be carried out by chemical means or enzymatically.
The proteoloytic cleavage site that is used to cleave off one of the affinity tags is preferably an enzymatic cleavage site. There are several proteases which are highly specific for short amino acid sequences which they will cleave. One of these is a specific cleavage site of Tobacco Etch Virus (TEV), which is cleaved by the TEV protease NIA. Recombinant TEV protease is available from Gibco BRL. The TEV cleavage site is preferably used as the cleavage site to remove the protein A domains from the fusion protein.

Even more preferably, the affinity step using protein A binding to IgG is carried out first by binding the one or more polypeptides or subunits via the one or more IgG binding domains of Staphylococcus to a support material capable of specifically binding the latter, removing substances not bound to the support material and separating the one or more polypeptides or subunits from the support material by cleaving off the IgG binding domains via the specific proteolytic cleavage site, and then another affinity tag is used to purify the protein further via a conventional elution step comprising binding the polypeptide or subunit via another affinity tag to a second support material capable of specifically binding the latter, removing substances not bound to the support material and separating the polypeptide or subunit from the support material.

When the proteins are present at low concentrations in the expression environment and on the support material, a large amount of protease is required to release the bound material from the support material. In other words, when the substrate concentration is low a high level of enzyme is required to drive an efficient proteolytic reaction. The second purification step is then important to remove remaining contaminants and the protease. Removal of the protease is preferable in order to eliminate any negative influences of the proteolytic activity on the preparation.

However, in some cases it may be desirable to remove all the affinity tags in which case it is also possible to utilise two or more different proteolytic cleavage sites for the separation of the polypeptide/subunit of interest from the support material.

The method according to the invention not only facilitates efficient purification of proteins of interest but also allows fishing for and detecting components present in complexes with which the polypeptides or subunits are associated or complexed either directly or indirectly, e.g. molecules such as linker mediators. This would allow selective fishing for certain substances which may be potential drugs even from complex mixtures.

According to a second embodiment of the invention it is possible not only to detect or purify the subunit containing fusion proteins expressed but also other substances that are capable of associating with the proteins expressed in a direct way, i.e. by directly binding to the fusion protein, or indirectly via other molecules to form biomolecule complexes. If a fusion protein of a subunit of a biomolecule or protein complex is purified according to the invention the affinity steps are chosen so that other complex components which have bound to the fusion protein are still associated with the subunit after the purification steps so that they can be detected/purified as well.

The biomolecule complexes can be formed in the expression environment such as cellular complexes. Alternatively, other complex components may be added to the subunits already expressed to form complexes in vitro or may even be added when the subunit containing fusion protein is already bound to a support material in an affinity step.

It is also possible to express two (or more) subunits of the same complex each as a fusion protein with a different affinity tag. When the subunits associate they can be detected/purified possibly together with other complex components by a series of affinity steps in which each time the complex is bound via a differently tagged subunit. The two or more affinity tags can be fused with a single subunit of a complex or with two or more subunits which bind to each other or are simply present in the same complex.

The purification steps can be carried out as described above.

Some polypeptides are present in more than one complex so that the components of all complexes can be purified.

If one is interested in a single complex A one can also subtract other complexes B that also contain one of the subunits of A by fusing that subunit of A to one tag and fusing a subunit unique to B with a different tag. The tagged subunit of B will bind to a specific support material. If the fraction not bound to that support material is used in the second affinity purification step, complex B will no longer be present because it was removed (subtracted) in the first affinity step. Many similar scenarios can be envisaged and designed by a person skilled in the art.

Further affinity tags in addition to the IgG binding domains that can be used in accordance with the present invention can be any conventional affinity tag. Preferably, the second affinity tag consists of at least one calmodulin binding peptide (CBP). Calmodulin binding peptide as an affinity tag has been described and is commercially available (Stratagene). When a calmodulin binding peptide is used the corresponding purification step is carried out using a support material that is coated with calmodulin. The calmodulin binding peptide tag binds to calmodulin in the presence of low concentrations of calcium. It can subsequently be eluted using a chemical agent such as a chelating agent like EGTA. Preferably, around 2 mM EGTA is added for the elution step.

Another aspect of the present invention is a fusion protein consisting of one or more polypeptides or subunits fused to at least two affinity tags, wherein one of the affinity tags consists of at least one IgG binding domain of Staphylococcus protein A.

Other fusion proteins according to the invention are those additionally including a proteolytic cleavage site, preferably to cleave off the IgG binding domains, or those in which the second tag represents one or more CBPs. Again, the skilled person will be able to select and construct the most suitable combinations of tags and cleavage sites and polypeptides and/or subunits in fusion proteins depending on the affinity strategy used. The fusion protein can be constructed so that the above-mentioned purification, detection or fishing procedures can be carried out.

There are several possibilities for constructing the fusion protein. In principle, the affinity tags may be fused close to either of the N- or C-terminal ends of the polypeptide(s) or subunit(s) to be expressed. The order in which the tags are fused with the polypeptide(s) or subunit(s) is not critical but can be chosen according to the affinity protocol to be used. Small peptides such as the CBP can even be fused to the polypeptide(s) of interest internally (as long as the reading frame on the nucleic acid is not changed). Preferably, the tags are located near to the same end of the polypeptide(s) or subunit(s), wherein it is especially preferred that the IgG binding domains are placed at the N- or C-terminus of the complete fusion protein, followed by a proteolytic cleavage site, the other tag(s) and the polypeptide(s) or subunit(s).

The fusion protein can also contain a second proteolytic cleavage site for the removal of the second affinity tag. The most preferable combination of affinity tags and cleavage sites is the one with protein A domains of Staphylococcus as the first affinity tag which can be cleaved off via the TEV protease and using at least one calmodulin binding peptide as the second affinity tag which allows the elution of the truncated fusion protein using a chelating agent such as 2 mM EGTA.

Another aspect of the present invention is a heterologous nucleic acid coding for a fusion protein as the one described above.

A further aspect of the invention is a vector comprising at least one heterologous nucleic acid coding for a fusion protein of the invention. This vector contains the nucleic acid under the control of sequences which facilitate the expression of the fusion protein in a particular host cell or cell-free system. The control sequences comprise sequences such as promotors, and, if necessary enhancers, poly A sites etc. The promoter and other control sequences are selected so that the fusion protein is preferably expressed at a basal level so that it is produced in soluble form and not as insoluble material. Preferably, the fusion protein is also expressed in such a way as to allow correct folding for the protein to be in a native conformation. Preferably, one or more selectable markers are also present on the vector for the maintenance in prokaryotic or eukaryotic cells. Basic cloning vectors are described in Sambrook et al., Molecular Cloning, Molecular Cloning, A Laboratory Manual, Cold Spring Harbor Laboratory Press, (1989). Examples of vectors are plasmids, bacteriophages, other viral vectors and the like.

In a preferred embodiment vectors are constructed containing pre-made cassettes of affinity tag combinations into which the nucleic acid coding for the polypeptide or subunit of interest can be inserted by means of a multiple cloning site such a polynucleotide linker. Thus, a vector according to the invention is also one which does not contain the coding sequences for the polypeptide(s) or subunit(s) of interest but contains the above-mentioned components plus one or more polynucleotide linkers with preferably unique restriction sites in such a way that the insertion of nucleic acid sequences according to conventional cloning methods into one of the sites in the polynucleotide linker leads to a vector encoding a fusion protein of the invention.

In a further preferred embodiment the vector comprises heterologous nucleic acid sequences in form of two or more cassettes each comprising at least one of different affinity tags one consisting of one or more IgG binding domains of Staphylococcus aureus protein A, and at least one polynucleotide linker for the insertion of further nucleic acids. Such a vector can be used to express two subunits of a protein complex, each tagged with a different tag.

Vectors according to the invention can be introduced into host cells stably or transiently, they can be present extrachromosomally or integrated into the host genome, and they can be used to produce recombinant cells or organisms such as transgenic animals.

Another object of the invention is a cell containing a heterologous nucleic acid or a vector of the invention. These cells can be prokaryotic or eukaryotic cells, e.g. bacterial cells, yeast cells, fungi or mammalian cells, and the vector or nucleic acid can be introduced (transformed) into those cells stably or transiently by conventional methods, protocols for which can be found in Sambrook et al.(supra).

Yet a further aspect of the invention is a reagent kit preferably comprising vectors as described above together with support materials for carrying out the affinity steps. The support materials carry moieties which are capable of specifically binding the affinity tags, for example, calmodulin-coated resin in the case of calmodulin binding peptide as the affinity tag or IgG-coated resins for affinity tags consisting of protein A domains. Additionally, such a kit may comprise buffers and other conventional materials for protein purification, especially for affinity chromatography. Further, the kit preferably provides at least one proteolytic agent such as a chemical agent capable of performing proteolysis or a protease and/or chemical agents such as chelating agents, wherein the protease is capable of proteolytically cleaving the fusion protein. When two proteolytic cleavage sites are used the kit will preferably contain two proteases.

The following Examples and Figures serve to illustrate the invention and its practical application, they are, however, not intended to limit the scope of the invention.
- Fig.1: shows a Coomassie stained gel depicting the fractionated proteins of a yeast RNA-protein complex. Proteins identified by mass spectrometry are labeled 1-24. Bands 1, 3, 8-11, 16-24 were expected in this complex. Bands 2, 4-7 represent proteins that are likely candidates for true complex component given their sequence. Bands 12-14 represent potential contaminants (ribosomal proteins). Band 15 is a trace amount of TEV protease that remained in this particular preparation. This is not generally the case (see Fig.2 for example). Bands 4-6 originate from the same gene and might represent alternative translation products or degradation products. Band 16 is a mixture and contains, in addition to a bona fide complex protein, a contaminating ribosomal protein.
- Fig.2: shows a Coomassie stained gel depicting the fractionated proteins moiety of the yeast U1 snRNP. The 10 specific proteins were identified by mass spectrometry. Compare with the silver stained gel obtained following a purification using a cap affinity step and a Ni-NTA column reported by Neubauer et al., (Proc.Natl.Acad.Sci USA 1997, 94, 385-390). Note in particular the low level of contaminants in this purification.
- Fig.3: shows a Coomassie stained gel in which purified U1 snRNP has been analysed using either the CBP binding/EGTA elution alone (lane 1), the Protein A binding/TEV elution alone (lane 3) or both steps (lane 7). Arrows on the right point to the U1 snRNP specific proteins. Lanes 2, 4 and 8 show the background signal obtained using each of the single steps or the two step procedure from an extract without tagged protein, demonstrating the requirement for two steps to get a pure material. Lane 6 is a molecular weight marker. Lane 7 is the TEV protease that can also be seen as an abundant contaminant (even though only the required amount of protease was used) in lanes 3 and 4. This demonstrates again the need for a second purification step.

### Examples

### Example 1

### Purification of protein complexes from yeast

A vector encoding a fusion of a yeast protein to the CBP-TEV-Protein A double tag was constructed using standard methods. The fusion protein is one subunit of a protein complex of yeast containing 24 subunits in total. The plasmid was transformed into yeast cells and a 2 L culture of cells expressing the protein was prepared. Proteins were extracted from the cultured cells using a French press. The complex was purified by binding to IgG-linked beads, eluting by TEV protease cleavage, binding of the eluted material on calmodulin containing beads followed by elution with EGTA. All steps were carried out at 0-4°C, excepted for TEV cleavage.

The first affinity step (IgG step) was performed as follows:
200 µl of IgG-Sepharose bead suspension (Pharmacia 17-0969-01) were washed in an Econocolumn with 5 ml of IPP 150-IgG buffer (10 mM Tris-CI pH 8.0, 150 mM NaCI, 0.1 % NP40). 10 ml extract, corresponding approximately to 2 L of yeast culture, were adjusted to IPP 150-IgG buffer concentrations in Tris-Cl pH 8.0, NaCl and NP40. This extract solution was mixed with the 200 µl of IgG-Sepharose beads and rotated in the Econocolumn for 2 hours. The unbound fraction was discarded and beads with bound material were washed first with 30 ml IPP 150-IgG buffer followed by 10 ml TEV cleavage buffer (10 mM Tris-CI pH 8.0, 150 mM NaCI, 0.1% NP40, 0.5 mM EDTA, 1 mM DTT).

The target protein was cleaved and released from the beads as follows. The washed Econocolumn was filled with 1 ml TEV cleavage buffer and 30 µl TEV protease and rotated in a 16°C incubator for 2 hours. The eluate was recovered by gravity flow.

The second affinity step (Calmodulin affinity step) was performed as follows:
The previous eluate was mixed with 3 ml of IPP 150-Calmodulin binding buffer (10 mM β-mercaptoethanol, 10 mM Tris-CI pH 8.0, 150 mM NaCI, 1 mM Mg-acetate, 1 mM imidazole, 2 mM CaCl₂, 0.1% NP40). The appropriate amount of CaCl₂ was further added to block the EDTA coming from the TEV cleavage buffer. This mix was rotated for 1 hour in an Econocolumn containing 200 µl of Calmodulin beads slurry (Stratagene 214303) previously washed with 5 ml IPP 150-Calmodulin binding buffer.

After washing with 30 ml of IPP 150-Calmodulin binding buffer, protein complexes were eluted with 5 successive additions of 200 µl of IPP 150-Calmodulin elution buffer (10 mM β-mercaptoethanol, 10 mM Tris-CI pH 8.0, 150 mM NaCI, 1 mM Mg-acetate, 1 mM imidazole, 2 mM EGTA, 0.1% NP40).

Samples were frozen in dry ice and stored at -80°C. Proteins were concentrated by TCA precipitation (A. Bensadoun and D. Weinstein (1976), Anal. Biochem. 70, 241). The proteins were detected by polyacrylamide gel electrophoresis with subsequent staining of the gel with Coomassie blue. The result of the protein purification, a gel of which is depicted in Fig.1 demonstrates that the strategy employed is highly efficient. All the expected protein subunits which number 24 in this case can be detected.

### Example 2

The same procedure was used for two other protein or protein-RNA complexes from yeast where all expected protein subunits were detected using the method of the invention. Those are the CBC (Cap Binding Complex) and the U1 snRNP. The purified U1 snRNP is depicted in Figures 2 and 3. The CBC complex has been shown to be still active and the purity was good in all cases. This method is relatively cheap and not very time-consuming, since it can be done in one day. Concerning the U1 snRNP, it is noteworthy that Neubauer et al. (Proc. Natl. Acad. Sci. USA 1997, 94, 385-390) carried out a purification of the same complex (U1 snRNP) extracted from 16 L of culture. The proteins of the complex of interest were then only visible by silver staining and several contaminants were still observed.

## Claims

1. Method for detecting and/or purifying substances selected from proteins, biomolecules, cell components, cell organelles and cells comprising the steps:
(a) providing an expression environment containing one or more heterologous nucleic acids encoding one or more polypeptides and/or one or more subunits of a biomolecule complex, the polypeptides or subunits being fused to at least two different affinity tags, one of which consists of one or more IgG binding domains of Staphylococcus protein A,
(b) maintaining the expression environment under conditions that facilitate expression of the one or more polypeptides or subunits in a native form as fusion proteins with the affinity tags,
(c) detecting and/or purifying the one or more polypeptides or subunits by a combination of at least two different affinity purification steps each comprising binding the one or more polypeptides or subunits via one affinity tag to a support material capable of selectively binding one of the affinity tags and separating the one or more polypeptides or subunits from the support material after substances not bound to the support material have been removed.

2. Method according to claim 1, wherein between the one or more polypeptides or subunits and one or more of the affinity tags a specific proteolytic cleavage site is present in the fusion protein which facilitates the removal of one or more of the affinity tags.

3. Method according to claim 2, wherein the specific proteolytic cleavage site is an enzymatic cleavage site.

4. Method according to claim 3, wherein the specific proteolytic cleavage site is the cleavage site for TEV protease NIA.

5. Method according to claim 2, 3 or 4, wherein the proteolytic cleavage site is used to cleave the polypeptide or subunit in step (c) from the IgG binding domain of Staphylococcus protein A bound to the support material.

6. Method according to claim 5, wherein the affinity purification of step (c) comprises:
(i) binding the one or more polypeptides or subunits via the one or more IgG binding domains of Staphylococcus to a support material capable of specifically binding the latter, removing substances not bound to the support material and separating the one or more polypeptides or subunits from the support material by cleaving off the IgG binding domains via the specific proteolytic cleavage site, and
(ii) binding the polypeptide or subunit via another affinity tag to a second support material capable of specifically binding the latter, removing substances not bound to the support material and separating the polypeptide or subunit from the support material.

7. Method according to claim 6, wherein step (ii) is carried out before step (i).

8. Method according to one of the previous claims, wherein the fusion protein contains a second specific proteolytic cleavage site for the removal of one or more of the other affinity tags.

9. Method according to one of the previous claims, wherein one of the affinity tags consists of at least one calmodulin binding peptide.

10. Method according to claim 9, wherein a chemical agent is used to separate the one or more polypeptides or subunits from the support material.

11. Fusion protein comprising at least one polypeptide or subunit of a protein complex fused to at least two different affinity tags, wherein one of the affinity tags consists of at least one IgG binding domain of Staphylococcus protein A.

12. Fusion protein according to claim 11, wherein it additionally contains a specific proteolytic cleavage site.

13. Nucleic acid coding for a fusion protein according to claim 11 or 12.

14. Vector comprising a nucleic acid according to claim 13 under the control of sequences facilitating the expression of a fusion protein according to claim 12 or 13.

15. Vector comprising heterologous nucleic acid sequences in form of one or more cassettes each comprising at least two different affinity tags one consisting of one or more IgG binding domains of Staphylococcus aureus protein A, and at least one polynucleotide linker for the insertion of further nucleic acids.

16. Vector comprising heterologous nucleic acid sequences in form of two or more cassettes each comprising at least one of different affinity tags one consisting of one or more IgG binding domains of Staphylococcus aureus protein A, and at least one polynucleotide linker for the insertion of further nucleic acids.

17. Cell containing a nucleic acid according to claim 13 or a vector according to claim 14.

18. Reagent kit comprising a nucleic acid according to claim 13 or a vector according to claim 14, 15 or 16 for the expression of a fusion protein according to claim 11 or 12 and support materials each capable of specifically binding one of the affinity tags.

19. Reagent kit according to claim 18 additionally comprising at least one chemical agent for separating one of the affinity tags from its support material and/or a specific chemical proteolytic agent and/or specific protease capable of cleaving the fusion protein.

20. Use of the method according to one of claims 1 to 10 for the detection and/or purification of substances capable of complexing with the fusion protein.

21. Use of the method according to one of claims 1 to 10 for the detection and/or purification of cells and/or cell organelles expressing the fusion protein on their surface.
